# EUROPEAN PATENT SPECIFICATION

(11) **EP 1 399 590 B1**
(45) Date of publication and mention of the grant of the patent: **09.08.2006**
(21) Application number: 02732953.1
(22) Date of filing: 18.06.2002
(51) Int. Cl.: C12Q 1/68

(54) **HAPPIER MAPPING**
HAPPIER MAPPING
CARTOGRAPHIE HAPPY MAPPING

(30) Priority: 18.06.2001 GB 0114853
(43) Date of publication of application: 24.03.2004
(73) Proprietor: MEDICAL RESEARCH COUNCIL, London W1B 1AL (GB)
(72) Inventor: DEAR, Paul, H. Medical Research Council Laboratory, Cambridge CB2 2QH (GB); THANGAVELU, Madan MRC Cancer Cell Unit, Hutchinson, Cambridge CB2 2XZ (GB); BANKIER, Alan Medical Research Council Laboratory, Cambridge CB2 2QH (GB)
(74) Representative: Maschio, Antonio
(86) International application number: PCT/GB2002/002763
(87) International publication number: WO 2002/103046

(56) References cited:
- EP-A- 0 534 858
- US-B1- 6 218 119
- DEAR PAUL H: "HAPPY mapping." PRACTICAL APPROACH SERIES, vol. 184, 1997, pages 95-123, XP009013094 1997 Oxford University Press; Oxford University Press, Inc. Walton Street, Oxford OX2 6DP, England; 198 Madison Avenue, New York, New York 10016, USA ISBN: 0-19-963631-1
- SMITH D R: "LIGATION-MEDIATED PCR OF RESTRICTION FRAGMENTS FROM LARGE DNA MOLECULES" PCR METHODS AND APPLICATIONS, COLD SPRING HARBOR, NY, US, vol. 2, no. 1, August 1992 (1992-08), pages 21-27, XP000874173 ISSN: 1054-9803
- DEAR P H ET AL: "HAPPY MAPPING: A PROPOSAL FOR LINKAGE MAPPING THE HUMAN GENOME" NUCLEIC ACIDS RESEARCH, OXFORD UNIVERSITY PRESS, SURREY, GB, vol. 17, no. 17, 12 September 1989 (1989-09-12), pages 6795-6807, XP000371654 ISSN: 0305-1048 cited in the application
- VOS P ET AL: "AFLP: A NEW TECHNIQUE FOR DNA FINGERPRINTING" NUCLEIC ACIDS RESEARCH, OXFORD UNIVERSITY PRESS, SURREY, GB, vol. 23, no. 21, 1995, pages 4407-4414, XP000939214 ISSN: 0305-1048
- KOVACS AND KLÖTING: "MAPPING OF QUANTITATIVE TRAIT LOCI FOR BODY WEIGHT ON CHROMOSOMS 1 AND 4 IN THE RAT" BIOCHEMISTRY AND MOLECULAR BIOLOGY INTERNATIONAL, ACADEMIC PRESS, LONDON, GB, vol. 44, no. 2, February 1998 (1998-02), pages 399-405, XP002104407 ISSN: 1039-9712
- VALLEJO R L ET AL: "NON-ASSOCIATION BETWEEN RFP-Y MAJOR HISTOCOMPATIBILITY COMPLEX-LIKE GENES AND SUSCEPTIBILITY TO MAREK'S DISEASE VIRUS-INDUCED TUMOURS IN 63X72F2 INTERCROSS CHICKENS" ANIMAL GENETICS, BLACKWELL SCIENTIFIC PUBLICATIONS, LONDON, GB, vol. 28, 5 October 1997 (1997-10-05), pages 331-337, XP002074409 ISSN: 0268-9146

## Description

The present invention relates to an improved method for mapping nucleic acid molecules, based on HAPPY mapping, which allows the rapid mapping of large numbers of arbitrarily-defined genome markers. The invention is applicable, for example, to nucleic acid molecules where no conventional markers are available.

HAPPY mapping is a method which has been developed for linkage mapping of the genome of any organism. It was first described using single haploid sperm as a DNA source by Dear *et al.* in 1989 (see Dear and Cook, (1989) NAR 17:6795) and later adapted to use multiple diploid cells as a DNA source, followed by DNA dilution and aliquotting into mapping panel members, each containing ideally 0.69 haploid equivalents, using which marker linkage can be assessed; the technique has been reviewed and employed in several publications (for example, Dear and Cook, (1993) NAR 21:13-20; Piper *et al.,* (1998) Genome Res. 8:1299-1307; and various references cited therein).

Fundamentally, HAPPY mapping involves the breaking of genomic DNA into fragments which are physically separated to provide a panel of samples, each containing an amount of DNA preferably equal to less than one haploid equivalent of the genome in question (and ideally 0.69 haploid equivalents if sampled from bulk genomic DNA). The samples are then screened for presence of a series of markers. Markers which are located close together in the genome will cosegregate to a greater extent than markers which are more distant in the genome. By analysis of a cosegregation table obtained with a marker panel, the order and spacing of the markers in the genome can be deduced.

The main applications for HAPPY mapping include genome and gene mapping, detection of strain diversity, population analysis, epidemiology, gene expression and the demonstration of phylogenetic and taxonomic relationships.

One of the main difficulties encountered in generating a HAPPY map is the identification of suitable markers in the genome. Applications of HAPPY mapping have involved first 'pre-amplifying' all markers in the mapping panel simultaneously using various techniques, and then screening the pre-amplified samples by PCR for pre-defined markers, using specific primers. In an early theoretical paper describing HAPPY mapping (Dear and Cook, (1989) NAR 17:6795) it was suggested that multiple products arising from low-stringency PCR amplification with short, arbitrary primers could serve as markers, eliminating the requirement for costly marker-specific primers.

Amplification-based nucleic acid scanning techniques driven by synthetic oligodeoxynucleotide primers of arbitrary sequence produce characteristic fingerprints capable of detecting sequence polymorphisms in anonymous nucleic acid templates (reviewed in Caetano-Anollés G (1996) Nature Biotechnology 14: 1668-1674; Caetano-Anollés G (1998) Arbitrary oligonucleotides: primers for amplification and direct identification of nucleic acids, genes and organisms. In: Molecular Approaches to Ecology and Evolution, DeSalle R, Schierwater B (eds), pp. 107-123. Birkhauser Verlag, Basel). The amplification of genomic DNA using at least one short primer usually results in multiple amplification products representing amplicons more or less randomly distributed throughout a genome (Livak KJ *et al.,* (1992) US Patent 5,126,239; Bassam BJ *et al.* (1995) US Patent 5,413,909). This observation led to the inception of three major techniques, randomly amplified polymorphic DNA (RAPD) (Williams *et al.,* (1990) Nucleic Acids 18: 6531-6535), arbitrarily primed PCR (AP-PCR) (Welsh J, McClelland M (1990) Nucleic Acids Res 18: 7213-7218), and DNA amplification fingerprinting (DAF) (Caetano-Anollés G *et al.,* (1991) Bio/Technology 9: 553-557). These methods became very popular because of their simplicity and wide applicability. A wide range of organisms have been studied and research reported in thousands of publications. Of these techniques, RAPD has been the most widely used, notwithstanding the fact that most products of RAPD and similar techniques are non-polymorphic; the polymorphic products which are of interest in genetic linkage mapping are in the minority.

Moreover, the use of random amplimers as markers is hampered by the extreme sensitivity of the non-stringent amplification reactions to variations in conditions.

An alternative form of arbitrary amplimer has also been used as a marker in genetic linkage mapping: the DNA to be tested is first digested with a restriction enzyme; linkers are then ligated to the fragments, and the products amplified using primers complementary to the linker sequence. If the primers contain additional bases at their 3' ends ('selective linker primers'), then only a subset of ligated fragments will be amplified. If sufficient additional bases are used, then the number of amplified products is small enough to be resolved by gel electrophoresis. Hence, different selective linker primers can be used singly or in combination to amplify different subsets of genomic fragments, each of which can serve as markers. This approach is more reproducible than the RAPD method, as the selective primers are used at high stringency.

To date, this approach has only been applied to genetic linkage analysis, where polymorphisms in the genome are reflected in differences in a minority of the amplified products ('arbitrary fragment-length polymorphisms' ; AFLPs). The AFLP method cannot be applied to radiation hybrid (RH) or clone-based mapping, because there is no way to distinguish amplimers arising from the 'donor' genome from those arising from the 'host'; hence, monomorphic markers cannot be mapped.

### Summary of the Invention

We have now developed a HAPPY mapping methodology which makes use of random amplimer markers and circumvents many of the innate drawbacks of such markers. The new approach combines the advantages of HAPPY mapping with amplification using ligated linker-based specific priming and selective internal priming to generate subsets of amplified fragments which may be mapped by gel electrophoresis or other conventional techniques. Since it relies upon haploid genome equivalents, amplified by polymerase using linker-based priming, we have referred to this method as HAPPIER mapping.

In a first aspect, therefore, the invention provides a method for nucleic acid analysis which comprises:
a) preparing a HAPPY mapping panel comprising a plurality of nucleic acid samples derived from a nucleic acid to be mapped, each member of the said panel containing a sampling of DNA fragments representing an amount equal by mass to 0.05 to 2 copies 2 copies or less of the nucleic acid to be mapped;
b) cleaving the nucleic acid in said samples to completion at a defined sequence therein to produce nucleic acid fragments;
c) ligating linkers to either end of the nucleic acid fragments;
d) globally amplifying the nucleic acid using linker-specific primers;
e) providing a repertoire of probes in which each probe comprises a first sequence that is complementary or identical to the sequence of the linker and to that part of the restriction site, if any, which is retained at the termini of the fragments produced by restriction digestion, and an adjacent second sequence which is different in each probe of the repertoire;
f) hybridising one or more probes from said repertoire to the samples and scoring the presence or absence of sequences complementary to said one or more probes in the sample.

The invention may be performed as a single-run procedure, or may incorporate iterative steps of sequential selection of target fragments comprising the desired markers. Thus, in a preferred embodiment, step f) comprises the steps of:
i) amplifying a plurality of specific nucleic acid fragments from each sample;
ii) subdividing the fragments amplified in i) into sub-samples;
iii) hybridising one or more probes having a higher specificity than the probes used in i) to the sub-sample;
iv) optionally, repeating steps i) and ii) iteratively; and
v) scoring the presence or absence of sequences complementary to said one or more probes in the sample.

Preferably, the one or more probes in step g) are used to amplify specific nucleic acid fragments from the samples, allowing detection of the amplified nucleic acids by conventional means, for example as described below.

Each mapping panel sample comprises a haploid genome equivalent, which may be derived from single haploid cells, or by dilution of bulk nucleic acid derived from somatic (diploid) cells, as described previously. As used herein, a "haploid genome equivalent" will be understood as 0.05 to 2 equivalents, by mass, of the nucleic acid to be mapped; ideally, it is about 0.69 genome equivalents. The nucleic acids are broken into large fragments, in which linkage is largely maintained, to allow aliquotting into samples to form a mapping panel. This first breakage is normally by radiation or shearing to ensure randomness, and is into relatively large fragments (several kb to Mb). It is this breakage which determines the fineness of the map (though *not* the complexity of the signal, since the 'haploid' aliquot will contain approximately the same amount of DNA regardless of the size of the fragments).

The mapping panel samples are then cleaved using a restriction endonuclease. The size of the fragments generated will be determined by the frequency of cutting of the endonuclease; the more frequent the cutting, the greater the complexity of the signal which will need to be deconvoluted. Advantageously, a restriction endonuclease which generates sticky ends at the cleavage site is used, in order to facilitate linker ligation.

This second breakage is into small fragments (typically less than 2kb) for ligation and amplification. It provides a tool to enable global and selective amplification of the DNA in the sample. Since the markers have already been segregated into mapping panel members prior to this second cleavage it typically does not influence the fineness of the resulting map.

The nucleic acid is advantageously DNA.

Advantageously, the linker is between 7 and 40 nucleotides in length, preferably 15 to 25 nucleotides in length. The linker itself, plus one or more adjacent bases in the fragment sequence itself, form the unique site to which a probe from the probe repertoire is able to hybridise, depending on the probe length selected. The sequence of the linker may be any desired sequence; in order to minimise the risk that the sequence occurs naturally in the genome being mapped, such a sequence is advantageously selected from alternative sources, such as linkers derived from a different genus, or is screened to determine that hybridisation does not occur.

In addition to the constant sequence corresponding to the linker, each probe comprises one or more adjacent bases 3' thereto. Any number of adjacent bases may be relied upon; the person skilled in the art will recognise that it is necessary to balance the requirements of specificity of hybridisation with the disadvantages in cost and handling of using very long probes. Advantageously, the adjacent second sequence in the probe consists of between 2 and 20 bases; preferably 2, 3, 4, 5, 6, 7, 8, 9 or 10 bases.

The invention allows the rapid mapping of monomorphic markers, which are randomly-generated by the subdivision of the haploid genome equivalent into samples and the use of one or more specific probes from the probe repertoire. In each sample, only specific nucleic acid fragments which possess termini complementary or identical to the specific probe used will be amplified. Analysis of the cosegregation of amplified fragments in the sample population will determine marker linkage and thus the map of the haploid genome.

In an advantageous embodiment, the use of one or more probes from a probe repertoire is replaced by the use of a fully sequence-specific probe to amplify a single fragment from each sample. This allows conventional STS (sequence-tagged-site) markers to be scored.

In a further aspect, the invention relates to a method for encoding the contents of a microtitre plate so that they can be tracked though further procedures and manipulations. The method of the invention allows microtitre plate contents to be tracked even if they are transferred or combined with samples in another plate, or loaded onto a gel.

In a first embodiment of this aspect of the invention, a solid-phase marker such as inert fluorescent microspheres (e.g., Molecular Probes A3703) is added to some of the wells in the plate; the pattern of wells thus labelled represents a unique "signature" of the plate contents. For example, the position of the markers can encode a binary number.

In a preferred embodiment, microspheres are added to a microtitre plate destined for PCR; the particles do not interfere with the reaction, and are loaded onto the gel when the samples are subsequently analysed. The 'code' of the microtitre plate is transferred to the gel - the wells containing the fluorescent particles light up when the gel is photographed under UV light. The particles do not migrate into the gel during electrophoresis. This overcomes a general problem in tracking samples in electrophoresis - namely that it is easy to print a barcode or similar on a microtitre plate, but difficult to transfer the coding to a gel. In an alternative embodiment, DNA is loaded into some of the unused wells of the gel to encode a binary number; however, this requires the user to read the number on the microtitre plate, and then arrange for the same number to be encoded on the gel. With the system according to the inventnion, the robot which sets up the PCRs can also add the fluorescent particles, and the 'code' is transferred to the gel upon loading, with no further intervention or opportunity for error.

The invention is moreover applicable to further embodiments. For instance, where reactions are prepared by taking one set of reagents from one plate (e.g. a set of PCR templates) and another set from another plate (e.g. a set of PCR primers), each source-plate can be encoded by spiking certain wells with the particles. Conveniently, the coding can occupy the first row of wells in one plate, and the last row in the other plate. The reaction plate (containing the contents of the two source plates) then bears both sets of encoding markers; by imaging the plate under UV illumination the codes can be seen and verified. Alternatively, the codes will be seen when the samples are analysed by gel electrophoresis.

Multiple colours can be used to provide more complex codes, or to allow two or more codes to be superimposed in the same set of wells.

### Detailed Description of the Invention

Although the general techniques mentioned herein are well known in the art, reference may be made in particular to Sambrook *et al.,* Molecular Cloning, A Laboratory Manual (1989) and Ausubel *et al.,* Short Protocols in Molecular Biology (1999) 4^{th} Ed, John Wiley & Sons, Inc (as well as the complete version Current Protocols in Molecular Biology).

### 1. Definitions

A "mapping panel" as referred to herein is a panel of nucleic acid fragments which have been separated into separate samples, or members. Each member of the panel may consist of some fraction (typically 1/2 or 1/3rd) of the fragmented DNA isolated from a single haploid cell, as in Dear & Cook (1989). More generally, each member may consist of a sample of fragmented DNA prepared from two or more haploid cells or from one or more diploid cells, and ideally containing an amount of DNA equal in mass to 0.69 genomes (i.e., 0.69 haploid equivalents); this amount ensures that, assuming a Poisson distribution of sequences sampled from bulk DNA, approximately half of all markers are represented in each sample; however, amounts of DNA between about 0.05 and 2 fall within the acceptable range.

The mapping panel used in the invention may be any mapping panel which contains DNA fragments derived from genomic DNA or any other source which it is intended to map. Advantageously, it comprises at least two members, and advantageously about 4, 8, 16, 32, 64, 96, 100, 110, 128, 256 or more members. The use of 96 members is convenient. Further members may be present as control samples.

"Cleaving at a defined sequence" is intended to indicate that the genome equivalent is subjected to sequence-specific cleavage, for example by cleavage enzymes such as restriction endonucleases, to produce nucleic acid fragments. The cleavage results in the cleaved "ends" of the nucleic acid having a defined sequence. The use of type III restriction enzymes, which cleave remotely from the recognition site, or of type I enzymes which cleave randomly, is not envisaged. However, the cleavage enzymes may be natural or artificial. Artificial cleavage enzymes may be, for example, ribozymes or zinc finger polypeptides suitably modified (see, for example, International patent application WO 00/20622).

Cleavage "to completion" indicates that substantially all of the sites which possess the cleavable sequence are cleaved. This ensures that fragments having the same sequence are presented uniformly across multiple samples.

"Amplification" refers to any process for multiplying nucleic acid strands *in vitro.* Preferably, the process is enzymatic and may be linear or exponential in character. An exemplary technique is PCR, which exponentially amplifies nucleic acid molecules. Alternative amplification techniques include Reverse transcriptase-PCR, which is used to amplify RNA targets (Wang, *et al.,* (1989). Proc *Natl Acad Sci USA* **86** (24), 9717-21). In this process, the reverse transcriptase enzyme is used to convert RNA to complementary DNA (cDNA), which can then be amplified using PCR. This method is particularly useful where it is intended to map ribonucleic acid samples, such as the genomes of RNA viruses.

Self-sustained sequence replication (3SR or NASBA) involves the isothermal amplification of a nucleic acid template *via* sequential rounds of reverse transcriptase (RT), polymerase and nuclease activities that are mediated by an enzyme cocktail and appropriate oligonucleotide primers (Guatelli, *et al.,* (1990) *Proc Natl Acad Sci USA* **87**(5), 1874-8). Enzymatic degradation of the RNA of the RNA/DNA heteroduplex is used instead of heat denaturation. RNase H and all other enzymes are added to the reaction and all steps occur at the same temperature and without further reagent additions. Following this process, amplifications of 10⁶ to 10⁹ have been achieved in one hour at 42°C.

The ligase chain reaction or ligation amplification system uses DNA ligase and four oligonucleotides, two per target strand (Wu, D. Y. & Wallace, R. B. (1989) *Genomics* **4**(4), 560-9). The oligonucleotides hybridise to adjacent sequences on the target DNA and are joined by the ligase. The reaction is heat denatured and the cycle repeated.

The invention moreover comprises the use of any amplification technique which is available to those skilled in the art. Such techniques include, but are not limited to, rolling circle amplification (Lizardi, *et al.,* (1998) *Nat Genet* **19**(3), 225-32) and strand-displacement amplification (SDA; Walker, *et al.* (1992). *Proc Natl Acad Sci USA* **89**(1), 392-6).

A "marker" is a nucleic acid sequence which may be identified in linkage studies, for example by PCR or hybridisation analysis. Advantageously it is substantially unique within the genome under analysis, such that identification thereof is unambiguous.

"Global" amplification refers to the amplification of nucleic acids irrespective of sequence. The procedure is known in the art in other contexts and may be referred to as "whole-genome" amplification or "whole-genome PCR". Advantageously, each nucleic acid in a population which is globally amplified is amplified to the same degree, to produce an amplified population in which each member is faithfully represented. In some instances, however, whole genome PCR may not result in amplification of all sequences, but in amplification of a defined subset thereof.

A "repertoire" of probes is a set of nucleic acid molecules which differ from each other in sequence. In the context of the present invention, the probes comprise a constant nucleic acid sequence, complementary or identical to the linker, and a variable region adjacent (3') thereto. The variable region may be wholly or partially randomised, or designed to have one or more specific sequences.

Depending on where within its recognition site the restriction enzyme cleaves, one or more bases from the site will be left on all fragment termini. In this case, the "constant" part of the probe will generally include not just the linker sequence (or its complement) but also the portion of the recognition site which is left on the relevant strand of the fragment termini.

"Resolving" the amplified fragments refers to resolution thereof, for example on the basis of length, such that each fragment is individually identifiable in each sample.

### 2. HAPPY mapping

General techniques for HAPPY mapping are well known in the art and have been extensively described in the literature. The following disclosures, which comprise detailed descriptions of HAPPY mapping, are incorporated herein by reference in their entirety: Konfortov *et al.,* Genome Res. 2000 Nov;10(11):1737-42; Williams and Firtel, Genome Res. 2000 Nov;10(11):1658-9; Piper *et al.,* Genome Res. 1998 Dec;8(12):1299-307; Lynch *et al.,* Genomics. 1998 Aug 15;52(1):17-26; Dear *et al.,* Genomics. 1998 Mar 1;48(2):232-41; Walter *et al.,* Nucleic Acids Res. 1993 Sep 25;21(19):4524-9; Dear and cook, Nucleic Acids Res. 1993 Jan 11;21(1):13-20; Dear and Cook, Nucleic Acids Res. 1989 Sep 12;17(17):6795-807. The present invention is based on HAPPY mapping, with several key differences.

A first difference is that the nucleic acid is fragmented by sequence-specific cleavage, subsequent to breakage by radiation or other physical means and to separation into separate aliquots. Techniques for selection and use of restriction endonucleases, which are preferred enzymes for use in the present invention, are known in the art, for example from Sambrook *et al. (Op. Cit.).*

Alternative techniques for nucleic acid cleavage may be used, as long as sequence-specific 5' and 3' ends are generated. These include PCR with primers of defined sequence, wherein the nucleic acid to be analysed is amplified using one or more primers in an amplification reaction. The primer or primers are capable of hybridising in a substantially sequence-specific manner to the nucleic acid to be analysed and to form a hybrid in which the primer chain is capable of enzymatic chain extension. Depending on the number of sites to which the primer hybridises, a number of fragments corresponding to genomic sequence are generated. Since the nucleic acid will normally be double-stranded, it should be denatured before primers can hybridise.

The invention preferably involves cleavage at a single sequence. However, cleavage at multiple sequences, for example using more than one restriction enzyme, is possible. Where cleavage is effected at multiple sequences, linkers may be added to one or both sets of "ends" left by the cleavage; if only one specificity of linker is used, only fragments having two ends cleaved at the same sequence complementary to that linker sequence will be amplified.

Moreover, the present invention employs linker ligation as a means for enabling specific fragment amplification. Again, such methods are known to those skilled in the art. The linker (or primers as defined elsewhere herein) is composed of units which are either nucleotides or nucleotide analogues. Generally speaking, a nucleotide analogue is a compound which is capable of being incorporated in a chain of nucleotide residues and which is capable of hybridising in a base-specific manner with a base of a complementary nucleic acid chain. Analogues useful in the present invention are moreover substrates for chain-extending enzymes.

A nucleotide analogue may be a modified nucleotide wherein the base is modified, for example so as to affect base-pairing properties; and/or wherein the sugar or backbone moiety is modified, for example as in the amide linked backbones of PNA; *and*/*or* wherein the phosphate moiety is modified.

Backbone-modified nucleic acids include methylphosphonates, phosphorothioates and phosphorodithioates, where both of the non-bridging oxygens are substituted with sulphur; phosphoroamidites; alkyl phosphotriesters and boranophosphates. Achiral phosphate derivatives include 3' - O' - 5' - S - phosphorothioate, 3' - S - 5' - O - phosphorothioate, 3' - CH₂ - 5' - O - phosphonate and 3' - NH - 5' - O - phosphoroamidate. Peptide nucleic acids replace the entire phosphodiester backbone with a peptide linkage.

Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without compromising affinity.

Modification of the heterocyclic bases preferably maintains proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

The linker is preferably 7-40 residues in length overall. Usually a short linker with 15-25 residues is used, but primers with up to 30 or up to 40 residues, or 10 or fewer residues, are also useful. After amplification, all amplimers will have the same sequence at both ends, the length of that sequence depending on the primer. After breaking the genomic DNA, the nucleic acids are aliquoted into samples, cleaved by restriction endonucelase digestion, ligated to the linkers and amplified using a linker-specific primer. Since all the nucleic acid fragments incorporate the linker at each end, all fragments are amplified.

The hybridisation procedure according to the invention advantageously entails ligation only to the 5' end of each strand in the double-stranded restriction fragments. However, variants of the present invention within the scope of the claims may be performed using alternative patterns of ligation, wherein linkers are attached to the 3' ends of the nucleic acid strands, or a mixture of both 5' and 3' ends.

After the global amplification step, a subset of the nucleic acid fragments in each sample is amplified using specific primers or probes. Under these circumstances, chain extension only takes place where the nucleotide residues at the 3' end of the primer accurately match those of the sample nucleic acid. 5' ends do not require to be perfectly hybridised, as long as the remainder of the primer is sufficiently strongly hybridised to prevent dissociation. Advantageously, the primers are 100% complementary to the linker/sample nucleic acid target.

The primers may incorporate a base analogue that promotes degenerate binding, by having the ability to base pair with two or three of the natural bases, or universal, by forming base pairs with each of the natural bases without discrimination. Such analogues may be used, in conjunction with conventional randomisation techniques, in the manufacture of the probes. However, it is preferred that the probes are highly sequence-specific in their binding and do not hybridise to degenerate sequences.

Nucleotides or nucleotide analogues for addition during the chain extension step may be labelled for ease of detection. Examples of suitable labels include radioisotopes, fluorescent moieties, haptens, and components of chromogenic or chemiluminescent enzyme systems.

Additionally, or alternatively, primers of defined sequence may be labelled using specific tags which allows them to be readily identified. Examples include tags having different masses, which are separable by mass spectrometry; molecular barcodes which may be "read" using appropriate detection instruments; combinations of fluorescent tags, which generate a specific signature emission; and the like.

The nature of the labelling will determine the best method for detection of the markers present in each sample. Where the fragments are unlabelled, or all similarly labelled, the amplified fragments are advantageously detected by gel electrophoresis, as in conventional HAPPY mapping.

However, use of specific labels allows fragments to be sorted otherwise, such as by FACS or mass spectrometry. See, for example, Griffin *et al.,* (1997) Nature Biotechnology 15:1368. Where the labels may be made specific for each primer, individual aliquots of the sample may be sorted for the presence of specific fragments without the need for amplification.

### 3. Nucleic acid cleavage

Cleavage of nucleic acids in a sequence-specific manner may be performed by any suitable method, including restriction endonuclease digestion as set out above. Alternative methods include sequence-specific cleavage of double helical DNA by triple helix formation (see H.E. Moser and P.B. Dervan (1985) Science 238, 645-650), the use of radioactive nucleotides (e.g. Karamychev *et al.,* J Nucl Med 2000; 41:1093-1101); synthetic restriction enzymes as described in US patent 6,018,058; CAP- and Fos-based molecules (http://www.cryst.bbk.ac.uk/PPS2/projects /hastie/dsc.htm); and the use of zinc finger polypeptides, as described in WO 00/20622.

### 4. Uses

The improved HAPPY mapping techniques of the invention may be applied to any mapping project. Thus, mapping of genomes or genomic DNA, such as chromosomes, has already been shown to be susceptible to the application of HAPPY mapping techniques and is susceptible to the application of the improved methods described herein. Further uses of the invention may become apparent to one skilled in the art on the basis of this description.

Preferred applications for the present, or indeed any, HAPPY mapping technology are set forth below.

### A. Haplotyping

As described above, the method of HAPPY mapping relies upon the random breakage and random sampling of genomic DNA to produce a set (panel) of samples containing (typically) sub-genomic amounts of DNA. Sequences (markers) which are often found together in the same members of the panel (i.e., which co-segregate) can be inferred to lie close together in the genome, compared to the average size of the fragments. Radiation hybrid mapping shares some features with HAPPY mapping, but breakage is achieved by irradiation of living 'donor' cells followed by fusion to uninadiated 'host' cells of a different species; some donor chromosome fragments are retained in the resulting hybrids, which are then analysed for their content of donor markers.

In normal use, the markers analysed by either method are monomorphic or, if they are polymorphic, the polymorphism is disregarded (both alleles of a marker being scored as one). However, if the alleles of a polymorphic marker are distinguished and scored independently on the mapping panel, then haplotype information (i.e., the linkage phase between the alleles of two or more markers in the diploid genome) may be determined.

Haplotype information is of considerable interest, particularly in the human genome, where SNP haplotype information is valuable for a number of applications, including but not limited to the association between polymorphisms (particularly single-nucleotide polymorphisms, SNPs) and susceptibility to disease or to adverse reactions to drugs, which is currently being researched extensively; the association of SNP haplotypes with 'normal' variable traits across a population; and the use of SNP haplotypes to trace human population movements.

HAPPY mapping may be applied to haplotyping as follows. A mapping panel is prepared in the usual way, but marker detection and scoring is performed in such a way as to discriminate between the alleles of polymorphic markers (the two alleles of a marker are denoted here by upper- and lower-case, for example A, a), and the results for each allele recorded independently. If two or more polymorphic markers are thus scored, then the proximity between the alleles of each may be determined. For example, if the parental genome contains haplotypes AB and ab, and if marker A/a lies sufficiently close to marker B/b, then cosegregation (and hence linkage) will be observed between A & B, and between a & b, but not between A & b or between a & B. Hence, the parental haplotypes may be determined. The distance across which haplotype information can be obtained is determined by the size of the fragments used in preparing the mapping panel.

Many methods exist for the necessary scoring and discriminating between alleles, depending upon the nature of the polymorphism. Any of these methods may be applied in this context.

In many instances, the location of the markers in the genome will already have been determined, and only the linkage phase is required. In these cases, fewer panel members need to be analysed than would be necessary to determine the order and spacing of the markers *a priori.* Also, where map information already exists, the resolution of the mapping panel becomes almost irrelevant; hence, the panel can be prepared from DNA fragments as large as possible to maximise the range over which haplotypes may be determined. In an extreme case, the genomic DNA can remain unbroken, with complete chromosomal DNA molecules (or chromosomes) being segregated amongst the panel members; in this instance, the results will be unable to determine the order or spacing of markers along a chromosome, but will yield haplotype data over chromosomal distances. Ideally, HAPPY panels prepared for use in haplotyping should contain approximately twice as much DNA as those used for routine mapping, since each allele is considered as an independent marker. However, the acceptable range of DNA concentrations for standard HAPPY panels is broad enough to accommodate haplotyping.

In principle, the same method can be used to obtain haplotype data from radiation hybrid panels, although the relative difficulty of preparing RH panels may make this less attractive if haplotypes from large numbers of individuals are required.

### B. Mapping with Chromosomes

As mentioned above, it may be advantageous to map, or derive haplotype information, using whole chromosomes instead of broken nucleic acid. Moreover, the use of larger DNA fragments allows HAPPY mapping, normally useful at medium-to-high resolution, to be extended to provide low resolution maps.

In HAPPY mapping, DNA is conventionally first isolated in relatively pure form (in solution or in a protective gel matrix) before breakage by mechanical or other means. However, the fragile nature of long DNA molecules makes it difficult to manipulate fragments more than a few million basepairs (Mb) long. Hence, linkage between markers can only be easily determined over distances up to a few Mb.

The present invention describes several applications based upon the sampling and analysis (at limiting dilution) not of 'naked' DNA fragments but of complete chromosomes, fragments of chromosomes or chromatin. The natural packaging of DNA in these forms makes it possible to isolate and manipulate larger fragments than when handling naked DNA, including complete chromosomes.

DNA is released from cells in the form of either chromatin or metaphase chromosomes. In both of these forms, the DNA is stabilised and compacted by association with histones and other proteins (and may, optionally, be further stabilised by other treatments, such as the partial fixation techniques used when preparing metaphase chromosomes for *in-situ* hybridisation or flow-sorting). Mechanical breakage is then used to break the chromatin/chromosomes into fragments, a solution of which is diluted and dispensed into a panel of samples, each containing approximately 0.05-2 genome equivalents of DNA (but preferably similar amounts of DNA in each member of the panel); ranges between about 0.2 and about 1.5 are generally considered useful. Use of above 2 copies is disadvantageous, but there is no rigid lower limit to the number of copies that can be used. The principle of the invention can be applied with arbitrarily low amounts of DNA per aliquot, provided that sufficient panel members are analysed to ensure that each marker sequence is represented in at least one (and preferably more than one) member. The samples are then analysed in the way already described for the analysis of HAPPY mapping panels; pre-treatment with proteinase-K or other methods may in some cases be required to ensure that the DNA becomes amenable to PCR amplification.

Since intact metaphase chromosomes are routinely prepared and handled in solution, it is clear that the fragments which are segregated into the panel members can be of any size, up to and including complete chromosomes. (It will be noted that, once segregation into samples is complete, further fragmentation of the DNA is of no consequence as long as its marker content is preserved.) The distances over which linkage can best be detected by HAPPY mapping are typically up to 0.5-0.7 times the average length of the DNA fragments used. Hence, a panel made from coarsely-broken chromosome fragments can be used to make sparsely-populated maps in which the average distance between markers is several Mb or more. This is useful in those genomes whose size makes it impracticable or uneconomic to make the very dense maps produced by conventional HAPPY mapping.

Fragments of metaphase chromosomes can also be flow-sorted (indeed, fragmented chromosomes are normally seen as a 'background' when flow-sorting chromosomes, and arise through unwanted degradation or shearing of the desired intact chromosomes). Hence, flow sorting may be used (instead of dilution and random sampling) as a method to segregate the required number and sizes of fragments into the members of the mapping panel. Such an approach has the advantages that (a) the total amount of DNA in each panel member can be finely controlled and (b) the size range of fragments can be narrowly selected; such tight selection allows the range and resolution of the panel to be fine-tuned to address the mapping problem in hand, and improves the quality of the mapping data by excluding fragments which are either too small to reflect linkage between any markers, or are so large as to contain no useful mapping information.

Intact metaphase chromosomes may be segregated into the members of the mapping panel, either by limiting dilution of a solution of such chromosomes or by flow sorting. In this case, the panel will give no information on the order and spacing of markers within one chromosome, but will allow markers to be co-localised in groups to their respective chromosomes. This is of particular value for chromosomally assigning markers in those species in which the chromosomes cannot be distinguished by flow cytometry. For example, chromosomes 9,10,11 and 12 of human cannot be distinguished by flow cytometry; if a mapping panel were prepared by flow-sorting one or two chromosomes (sampled at random from the Chr9-12 cluster) into each panel member, then the typing of markers on the panel would quickly allow the markers to be assigned to chromosomal linkage groups.

All of the above methods may also be applied to determining haplotypes (the linkage phase between polymorphic loci). In such cases, it is necessary only to score the two alleles of each marker independently (using established techniques for discriminating between alleles); then, each allele can be treated as an independent marker, and (for example) linkage will be observed between A & B, and between a & b, revealing the haplotypes AB and ab. The use of chromatin/chromosome fragments or intact chromosomes in making the mapping panels allows haplotypes to be determined over considerable (or chromosomal) distances.

The invention is further described below, for the purposes of illustration only, in the following example.

### Example

### Generation and pre-amplification of mapping panel

Nuclei are isolated from leaf cells of barley *(Hordeum vulgare,* variety 'Optic') and embedded in agarose strings (0.5% w/v low melting point agarose, -4000 nuclei per microlitre). The strings are immersed in lysis solution (0.5 M EDTA, pH 9.0, 1% lauryl sarcosine sodium salt, 0.1 mg mL⁻¹ proteinase K) and incubated at 45°C for 48 h with gentle mixing; then in 0.5 M EDTA, pH 9.0 for 1hr at 45°C; then in 0.05 M EDTA, pH 8.0 for 1hr on ice; and stored in 0.05 M EDTA, pH 8.0 at 4°C until needed. During this manipulation, the lysis solution diffuses into the agarose, lysing the nuclei and removing/degrading proteins and other nuclear material which diffuse out during the washing stages, leaving substantially pure DNA trapped within the agarose. The agarose serves to protect the DNA from unwanted mechanical breakage, since it is very important that the frequency of breaks in the DNA is controlled.

Breaks are introduced by melting a short section of string in 5ml of magnesium-free PCR buffer [this is used in preference to water, to reduce the risk of denaturing the DNA] at 68°C, and inverting the tube several times to both disperse the DNA and shear it into fragments of around 50-100kb. The solution is allowed to cool and diluted with water to a concentration of approximately 0.1 haploid genome equivalents per microlitre, using wide-bore pipette tips to avoid further mechanical shearing of the DNA. 5µl samples of this solution are dispensed into 88 wells of a 96-well microtitre plate, and 5µl of water into each of the remaining 8 wells. The samples are each overlaid with one drop (~30µl) of light mineral oil to prevent evaporation.

A digestion master mix is prepared comprising, per 2µl volume: 0.7µl of One-Phor-All buffer (Pharmacia; a general-purpose buffer suitable for a number of reactions), 4.2 units of restriction enzyme *Dpn*II (New England Biolabs), and water. 2µl of this mix is dispensed into each well of the microtitre plate. The plate is centrifuged briefly (~500g for 5 seconds) to ensure that all aqueous components unite beneath the oil overlay in each well. The microtitre plate is incubated at 37°C for ten minutes. The restriction enzyme is then inactivated by heating to 65°C for 30 minutes. *Dpn*II cleaves at the recognition sequence GATC, and conditions are chosen such that cleavage proceeds to completion.

A ligation master mix is prepared comprising, per 6µl volume: 1.5µl of 100µM solution of oligo LIB1, 1.5µl of 100µM solution of oligo dd-Sau3A, 0.8µl of One-Phor-All buffer, 0.26µl of 1M NaCl, and water. 6µl of this master mix is added to each well of the microtitre plate, which is centrifuged briefly as before. The plate is incubated at 85°C for 20 minutes, and then cooled at 1°C per minute to a temperature of 15°C. The oligonucleotides are:
LIB1 5'AGT GGT ATT CCT GCT GTC AGG 3'
ddSau3A 5' GAT CCC TGA CAG C* 3'
where C* indicates a di-deoxy nucleotide.

A ligase master mix is prepared comprising, per 2 µl volume: 1 µl of T4 DNA ligase (5 units; Boehringer-Mannheim), and 1µl of 15mM rATP (buffered in Tris-HCl at pH 7.4). 2µl of this solution is added to each well, and the plate is again centrifuged briefly to amalgamate the aqueous components underneath the oil overlay. The plate is then incubated at 16°C overnight (~16 hours).

The oligonucleotides are arranged as follows:

5' AGTGGTATTCCTGCTGTCAGGGATCXXXXXXXXXXXXXXXGATCCCTGACAGC* 3'
3' *CGAGAGTCCCTAGYYYYYYYYYYYYYYYCTAGGGACTGTCGTCCTTATGGTGA
5'

Italic=LIB1, undeline=ddSau3A, normal=restriction fragment (X,Y=any bases; length typically 100-500bp). NOTE - ligation occurs only between. LIB 1 and the restriction fragment (indicated by doubleunderline) and NOT between ddSau3A and the fragment (since the necessary phosphate groups are not present). For the same reason, there can be no ligation between ddSau3A and LIB1 oligos alone, which would interfere with subsequent amplifications ("linker dimer").

A whole-genome amplification mix is prepared comprising, per 37µl volume: 5µl of 10 PCR Buffer No 1 (Boehringer Mannheim), 0.8µl of 25mM dNTP solution (ie, 25mM each of dATP, dCTP, dGTP, dTTP), 1.4µl of Expand Long Polymerase (Boehringer Mannheim), and water. 37µl of this solution is dispensed into each well of the microtitre plate. Amplification of all restriction fragments [ideally - certain fragments may fail to amplify, because for example they are too large, lying in a region of sequence with widely-spaced recognition sites for *Dpn*II] is then achieved by thermocycling as follows:
1) 68°C x 4 min [during this step, oligo ddSau3A is denatured, and extension of the 3' end of each strand of each restriction fragment takes place to synthesise a region complementary to the ligated LIB 1 oligo; after this step, therefore, all restriction fragments carry the LIB 1 sequence and its complement at each end).
2) 14 cycles of: 94°C x 40sec, 57°C x 30 sec, 68°C x 75sec
3) 34 cycles as above, but 68°C for 105sec rather than 75 sec
4) 1 cycle as above, but 68°C for 300sec rather than 75sec
(During steps 2, 3 and 4, exponential amplification of the linked restriction fragments occurs, primed by the surplus of oligo LIB1).

The reactions are then diluted serially to 1:8000 in water, and stored at -20°C until required for either arbitrary or specific marker detection (below).

### Detection of specific markers

For the detection of specific markers (ie, of pre-determined sequences using specific primers), 5µl of each of the diluted products from above are amplified in a reaction comprising (in addition):
1x 'Gold' PCR buffer (Perkin Elmer)
0.25U 'Gold' DNA polymerase
1.5mM MgCl2
1µM each of specific forward and reverse oligonucleotides (see below for examples)
200µM of each dATP, dCTP, dGTP, dTTP
Total volume 10µl

Reactions are set up in 96-well microtitre plates and either overlaid with mineral oil (±30µl per well), or sealed using an appropriate sealing film. Thermocycling is performed as follows:
93°C x 5min
38 cycles of: 94°C x 20sec, 55°C x 30sec, 72°C x 60 sec
(The annealing temperature, 55C in the above example, may be adjusted according to the melting temperatures of the oligonucleotide primers).

Products are supplemented with a suitable loading buffer (8µl of 15% w/v Ficoll 400, 0.15mg/ml bromophenol blue, 4x SyBr Green, in 1x TBE buffer) and 10µl samples of the mixture are analysed by electrophoresis (6% polyacrylamide gel in 0.5x TBE buffer).

For example, the above procedure was performed successively for three specific sequences ('markers') whose position wihin the Barley genome is already known (and which could therefore serve as a test of the system). The specific primer sequences are:
14-4:
   Forward=GTCACTTGTCATCATTTGTCC
   reverse=GCACCATGAATACAATCATCC
14-5:
   Forward=CAACGATGAGATGGTAACCG
   reverse=CTCGCAGTCTGTTCGTTGG
1-16B: Forward=CTGTGCAAACAACATGACC,
   reverse=CTGTTTGACCAGTTGTTTGC

Each primer pair amplifies a short (few hundred base-pair) segment of known DNA sequence; the segments are in each case known not to contain within them a restriction site for *Dpn*II*.* Markers 14-4 and 14-5 are known to lie very close to one another (<2kb), whilst 1-16B is known to lie at a great distance (>100kb) from these two.

Analysis of the results shows that there was strong co-segregation between 14-4 and 14-5, the two markers each being present in ±60% of the 88 members of the mapping panel (excluding the 8 negative controls, which contained no markers), and with great similarity in the distribution of these two markers across the panel. A Lod score of 14.2 is calculated between these two markers, this being highly indicative of linkage. Marker 1-16B is also present in ±60% of the non-negative-control members of the mapping panel, but its distribution shows no obvious correlation to that of the other two markers; the Lod scores between 1-16B and 14-4, and between 1-16B and 14-5, are 0.4 and 0.7 respectively, these being non-significant values.

It will be apparent that the diluted product of the ligation-mediated PCR is sufficient to type approximately 80,000 specific markers in this way. However, it is also apparent that this product is susceptible to further non-selective amplification using the LIB 1 primer, providing essentially unlimited amounts of material.

### Detection of Arbitrary markers

A 5µl fraction is taken from each of the diluted pre-amplification products, and supplemented with reagents to give a total reaction volume 10µl containing (in addition to the diluted products), 0.25 Units of thermostable DNA polymerase (Taq Gold, Perkin Elmer), Ix PCR 'Gold' buffer (recommended by the supplier of the polymerase), 1.5mM magnesium chloride, 200µM of each dNTP and 1µM of primer LIBSEL-A (5' CCT GCT GTC AGG GAT CGT CC 3').
(The first 12 bases are identical to the 3' twelve bases of LIB1; the next four are identical to the recognition sequence of DpnII, which is common to all fragments; the last four bases are selective, having counterparts only in ~1/256th of fragment termini). The mixture is overlaid with mineral oil to restrict evaporation, and subjected to thermocycling (93°C x 9min; then 33 cycles of 94°C x 20sec, 64°C x 30sec, and 72°C x 60sec). The products of each reaction are analysed by gel electrophoresis using standard protocols, capable of resolving fragments having sizes of between 100 and 500 bases.

Each such reaction (corresponding to each member of the mapping panel) yields a number of products, the products in each case being a subset of those few tens of *Dpn*II fragments of the genome which carry the appropriate four selective nucleotides internally to the restriction site at each end. Each reaction is scored for the presence or absence of each of the resolvable fragments, and the fragments mapped relative to each other by seeing how often they co-segregate in the 96 aliquots. Each fragment can therefore be considered to be a marker, defined by a combination of its size (as determined by the electrophoresis), the restriction enzyme used to generate it (*Dpn*II) and the selective primer (LIBSEL-A) used to amplify it. Some markers cannot be mapped, either because the amplifications (global or selective) fail for any of various reasons or because the marker is not a single copy sequence (easily seen by noting the number of positives in the 96 aliquots) or because it is of a size too similar to another marker and therefore cannot be resolved during the electrophoresis. The majority of the markers, however, can be detected in a proportion of the samples and mapped by tabulation and calculation of linkage frequencies.

## Claims

1. A method for nucleic acid analysis which comprises:
a) Providing a HAPPY mapping panel comprising a plurality of nucleic acid samples; each sample containing randomly-selected fragments of broken DNA from the nucleic acid to be mapped, and amounting in mass to between 0.05 and 2 times the mass of a single copy of the nucleic acid to be mapped;
b) cleaving the nucleic acid in said samples to completion at a defined sequence therein to produce nucleic acid fragments;
c) ligating a linker to the 5' and 3'ends of the nucleic acid fragments;
- d) globally amplifying the nucleic acid using linker-specific primers;
e) providing a repertoire of probes in which each probe comprises a first sequence that is complementary or identical to the sequence of the linker, and an adjacent second sequence which is different in each probe of the repertoire; and
f) hybridising one or more probes from said repertoire to the samples and scoring the presence or absence of sequences complementary to said one or more probes in the sample.

2. A method according to claim 1, wherein step d) comprises the steps of:
i) amplifying a plurality of specific nucleic acid fragments from each sample;
ii) subdividing the fragments amplified in i) into sub-samples;
iii) hybridising one or more probes having a higher specificity than the probes used in i) to the sub-sample;
iv) optionally, repeating steps i) and ii) iteratively; and
v) scoring the presence or absence of sequences complementary to said one or more probes in the sample.

3. A method according to claim 1 or claim 2, wherein the one or more probes in step e) are used to amplify specific nucleic acid fragments from the samples.

4. A method according to any preceding claim, wherein two or more samples of the mapping panel are derived from a single haploid cell.

5. A method according to any one of claims 1 to 3, wherein the samples of the mapping panel are derived from one or more diploid cells, or two or more haploid cells, by dilution.

6. A method according to any preceding claim wherein the mapping panel samples are cleaved using one or more restriction endonuclease enzymes.

7. A method according to any preceding claim, wherein the linker is between 7 and 40 nucleotides in length.

8. A method according to any preceding claim, wherein each probe in the probe repertoire comprises the linker sequence or a sequence complementary thereto, and a further sequence of between 2 and 20 nucleotides.

9. A method according to claim 8, wherein said further sequence is an at least partially randomly generated sequence.

10. A method according to claim 8, wherein each probe is of known sequence.

11. A method according to any preceding claim, wherein the presence or absence of any nucleic acid fragment in each sample is scored by gel electrophoresis.

## Patentansprüche

1. Verfahren für die Nukleinsäureanalyse, welches folgendes umfaßt:
a) Bereitstellen eines HAPPY-Kartierungselements, das eine Vielzahl von Nukleinsäureproben umfaßt, wobei jede Probe zufällig ausgewählte Fragmente gebrochener DNA von der zu kartierenden Nukleinsäure enthält und eine Masse zwischen dem 0,05- und 2-fachen der Masse einer einzelnen Kopie der zu kartierenden Nukleinsäure aufweist,
b) Spalten der Nukleinsäure in den Proben bis zur Vollständigkeit an einer definierten Sequenz darin unter Erzeugung von Nukleinsäurefragmenten,
c) Ligieren eines Linkers an die 5'- und 3'-Enden der Nukleinsäurefragmente,
d) umfassendes Vervielfältigen der Nukleinsäure unter Verwendung von linkerspezifischen Primern,
e) Bereitstellen eines Repertoires von Sonden, in denen jede Sonde eine erste Sequenz, die zu der Sequenz des Linkers komplementär oder identisch ist, und eine benachbarte zweite Sequenz, die in jeder Probe des Repertoires verschieden ist, umfaßt, und
f) Hybridisieren einer oder mehrerer Sonden aus dem Repertoire an die Proben und Registrieren des Vorhandenseins oder der Abwesenheit von Sequenzen, die zu der einen oder den mehreren Sonden in der Probe komplementär sind.

2. Verfahren nach Anspruch 1, wobei Stufe d) die Stufen umfaßt, in denen man
i) eine Vielzahl von spezifischen Nukleinsäurefragmenten aus jeder Probe vervielfältigt,
ii) die in i) vervielfältigten Fragmente in Unterproben aufteilt,
iii) eine oder mehrere Sonden mit einer höheren Spezifität als die in i) verwendeten Sonden an die Unterprobe hybridisiert,
iv) wahlweise die Stufen i) und ii) schrittweise wiederholt und
v) das Vorhandensein oder die Abwesenheit von Sequenzen, die zu der einen oder den mehreren Sonden in der Probe komplementär sind, registriert.

3. Verfahren nach Anspruch 1 oder Anspruch 2, wobei die eine Sonde oder die mehreren Sonden in Stufe e) zur Vervielfältigung von spezifischen Nukleinsäurefragmenten aus den Proben verwendet wird/werden.

4. Verfahren nach einem der vorangegangenen Ansprüche, wobei zwei oder mehr Proben des Kartierungselements von einer einzelnen haploiden Zelle abgeleitet sind.

5. Verfahren nach einem der Ansprüche 1 bis 3, wobei die Proben des Kartierungselements von einer oder mehreren diploiden Zellen oder von zwei oder mehreren haploiden Zellen durch Verdünnung erhalten wurden.

6. Verfahren nach einem der vorangegangenen Ansprüche, wobei die Kartierungselementproben unter Verwendung eines oder mehrerer Restriktionsendonukleaseenzyme gespalten werden.

7. Verfahren nach einem der vorangegangenen Ansprüche, wobei der Linker zwischen 7 und 40 Nukleotide lang ist.

8. Verfahren nach einem der vorangegangenen Ansprüche, wobei jede Sonde in dem Sondenrepertoire die Linkersequenz oder eine dazu komplementäre Sequenz und eine weitere Sequenz mit zwischen 2 und 20 Nukleotiden umfaßt.

9. Verfahren nach Anspruch 8, wobei die weitere Sequenz eine wenigstens teilweise zufällig erzeugte Sequenz ist.

10. Verfahren nach Anspruch 8, wobei jede Sonde eine bekannte Sequenz hat.

11. Verfahren nach einem der vorangegangenen Ansprüche, wobei das Vorhandensein oder die Abwesenheit irgendeines Nukleinsäurefragments in jeder Probe durch Gelelektroforese registriert wird.

## Revendications

1. Méthode d'analyse d'acides nucléiques, qui comprend les étapes consistant à :
a) fournir un ensemble de cartographie HAPPY comprenant une pluralité d'échantillons d'acides nucléiques, chaque échantillon contenant des fragments, sélectionnés de manière aléatoire, d'ADN dissocié provenant de l'acide nucléique à cartographier, et représentant en masse 0,05 à 2 fois la masse d'une copie unique de l'acide nucléique à cartographier ;
b) cliver l'acide nucléique présent dans lesdits échantillons totalement jusqu'à une séquence définie dans cet acide nucléique pour produire des fragments d'acide nucléique ;
c) réunir par ligation un segment de liaison aux extrémités 5' et 3' des fragments d'acide nucléique ;
d) amplifier globalement l'acide nucléique en utilisant des amorces spécifiques du segment de liaison;
e) fournir un répertoire de sondes dans lequel chaque sonde comprend une première séquence qui est complémentaire de ou identique à la séquence du segment de liaison, et une seconde séquence adjacente qui est différente dans chaque sonde du répertoire ; et
f) hybrider une ou plusieurs sondes provenant dudit répertoire aux échantillons et évaluer numériquement la présence ou l'absence de séquences complémentaires de ladite ou desdites sondes dans l'échantillon.

2. Méthode suivant la revendication 1, dans laquelle l'étape d) comprend les étapes consistant à :
i) amplifier une pluralité de fragments d'acides nucléiques spécifiques provenant de chaque échantillon ;
ii) subdiviser les fragments amplifiés en i) en échantillons secondaires ;
iii) hybrider une ou plusieurs sondes ayant une plus forte spécificité que les sondes utilisées en i) à l'échantillon secondaire ;
iv) facultativement, répéter les étapes i) et ii) de manière itérative ; et
v) évaluer numériquement la présence ou l'absence de séquences complémentaires de ladite ou desdites sondes dans l'échantillon.

3. Méthode suivant la revendication 1 ou la revendication 2, dans laquelle la ou les sondes dans l'étape e) sont utilisées pour amplifier des fragments d'acide nucléique spécifiques à partir des échantillons.

4. Méthode suivant l'une quelconque des revendications précédentes, dans laquelle deux ou plus de deux échantillons de l'ensemble de cartographie sont dérivés d'une cellule haploïde unique.

5. Méthode suivant l'une quelconque des revendications 1 à 3, dans laquelle les échantillons de l'ensemble de cartographie sont dérivés d'une ou plusieurs cellules diploïdes, ou bien de deux ou plus de deux cellules haploïdes, par dilution.

6. Méthode suivant l'une quelconque des revendications précédentes, dans laquelle les échantillons de l'ensemble de cartographie sont clivés en utilisant une ou plusieurs enzymes consistant en endonucléases de restriction.

7. Méthode suivant l'une quelconque des revendications précédentes, dans laquelle le segment de liaison a une longueur de 7 à 40 nucléotides.

8. Méthode suivant l'une quelconque des revendications précédentes, dans laquelle chaque sonde dans le répertoire de sondes comprend la séquence du segment de liaison ou une séquence complémentaire de celle-ci, et une séquence supplémentaire de 2 à 20 nucléotides.

9. Méthode suivant la revendication 8, dans laquelle ladite séquence supplémentaire est une séquence engendrée de manière au moins partiellement aléatoire.

10. Méthode suivant la revendication 8, dans laquelle chaque sonde a une séquence connue.

11. Méthode suivant l'une quelconque des revendications précédentes, dans laquelle la présence ou l'absence de n'importe quel fragment d'acide nucléique dans chaque échantillon est évaluée numériquement par électrophorèse sur gel.
